# FASCICULE DE BREVET EUROPEEN

(11) **EP 1 248 630 B1**
(45) Date de publication et mention de la délivrance du brevet: **14.07.2004**
(21) Numéro de dépôt: 00990076.2
(22) Date de dépôt: 20.12.2000
(51) Int. Cl.: A61K 31/70, A61K 7/48

(54) **UTILISATION D'UNE COMPOSITION DERMATOLOGIQUE CONTENANT DES OLIGOSACCHARIDES POUR LE TRAITEMENT DES MALADIES DE LA PEAU**
VERWENDUNG EINER OLIGOSACCHARIDE ENTHALTENDEN DERMATOLOGISCHEN ZUBEREITUNG ZUR BEHANDLUNG VON HAUTERKRANKUNGEN
USE OF A DERMATOLOGICAL COMPOSITION CONTAINING OLIGOSACCHARIDES FOR THE TREATMENT OF SKIN DISEASES

(30) Priorité: 20.12.1999 FR 9916060
(43) Date de publication de la demande: 16.10.2002
(60) Demande divisionnaire: 04075969.8
(73) Titulaire: Laboratoires G Pharm, 34980 St. Clément de Rivière (FR)
(72) Inventeur: MARTINEZ, Gérard, F-34980 St Clément de Rivière (FR); FRANCISCO, Christian, F-66250 St Laurent de la Salanque (FR)
(74) Mandataire: Warcoin, Jacques
(86) Numéro de dépôt international: PCT/FR2000/003607
(87) Numéro de publication internationale: WO 2001/045713

(56) Documents cités:
- EP-A- 0 652 012

## Description

Le domaine de la présente invention est celui des symptômes désagréables associés ou non à l'allergie et en particulier à l'asthme, l'eczéma, le prurit, le psoriasis, la conjonctivite allergique, l'urticaire, les réactions aux piqûres d'insectes, les démangeaisons en particulier les démangeaisons symptomatiques des grands brûlés.

Les propriétés de dégranulation des polynucléaires et notamment des éosinophiles, basophiles et mastocytes sont connues pour être impliquées dans les phénomènes d'allergies.

Ainsi, les basophiles matures du sang présentent des granules distribués au hasard et bordés par une membrane, ces granules contiennent divers produits (héparine, SRS-A, ECF-A) qui sont libérés lorsqu'un stimulus approprié induit une dégranulation. Ce stimulus est habituellement un allergène qui apparie les IgE spécifiques fixées à la surface de la cellule par les récepteurs appropriés. Les produits libérés par la dégranulation sont responsables d'une partie des symptômes désagréables associés à l'allergie, mais ils sont également impliqués dans l'immunité anti-parasitaire.

EP 652 012 décrit un très grand nombre de combinaisons sucre + acide aminé pour le traitement de différentes conditions, mais ne décrit pas spécifiquement la combinaison raffinose of arginine. En outre, les combinaisons sucre + acide aminé de EP 652 012 sont destinées à être ingérées, et non à être administrées par voie topique externe où par inhalation.

Les principaux composés de l'art antérieur connus pour leurs propriétés anti-allergiques sont les composés de la famille des anti-histaminiques telle que la chlorpheniramine (polaramine). Ces composés ne sont pas dépourvus d'effets secondaires tels que des risques de somnolence. C'est pourquoi la présente invention propose l'utilisation d'une association de composés qui sont dépourvus des effets secondaires des anti-histaminiques classiques et qui permettent néanmoins de traiter les allergies et autres affections ou désagréments du même type.

La Demanderesse a mis en évidence que l'utilisation de certains oligosaccharides est efficace pour réduire ou même bloquer les symptômes désagréables associés ou non à l'allergie et en tant qu'anti-inflammatoire.

Sans vouloir être liée à une quelconque théorie, il apparaît que la Demanderesse a montré que certains oligosaccharides possèdent une activité anti-dégranulante, anti-granulantè ou anti-activatrice d'une cellule cible de l'allergie et notamment du basophile humain stimulé selon une réaction IgE dépendante.

D'autres cellules spécialisées de la peau (comme par exemple les neurones qui par libération de substance P sont responsables de la douleur mais aussi induisent une dégranulation des basophiles) sont aussi concernés par cette activité.

Le phénomène de dégranulation ainsi que les symptômes désagréables qui y sont liés sont connus de l'homme du métier cependant les inventeurs de la présente demande n'excluent pas que l'association utilisée au sens de la présente demande intervienne en amont de la dégranulation : lors de la maturation des granules. C'est pourquoi, selon les inventeurs, cette association pourrait aussi posséder une activité dite « anti-granulante ».

La présente invention a trait à l'utilisation d'une association d'au moins deux composants pour la fabrication d'une composition pour le traitement d'au moins un symptôme choisi dans le groupe constitué par l'asthme, l'eczéma, le prurit, le psoriasis, la conjonctivite allergique, l'urticaire et les réactions aux piqûres d'insectes, les démangeaisons et en particulier les démangeaisons symptomatiques des grands brûlés ou à titre d'anti-inflammatoire, destinée à être administrée par voie topique externe ou par inhalation, telle que le premier composant - le principe actif - est un oligosaccharide de formule 1 contenant de trois à cinq unités osidiques et possédant au moins une unité D-galactose liée par une liaison α[1-6] avec une unité sucrose : 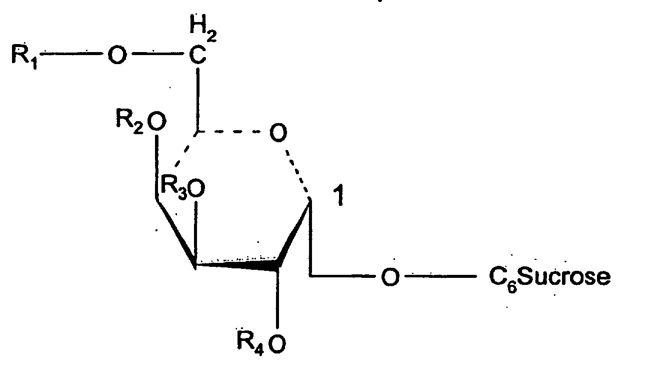 dans laquelle R₁, R₂, R₃ et R₄, indépendamment les uns des autres représentent un atome d'hydrogène, un groupe alkyle contenant de 1 à 10 atomes de carbone et possédant éventuellement au moins une insaturation, une fonction acide contenant de 1 à 10 atomes de carbone et possédant éventuellement au moins une insaturation, une fonction sulfate ou encore un ose, ledit oligosaccharide étant de préférence choisi dans le groupe constitué par le raffinose et le stachyose sous forme libre ou dérivée, et le deuxième composant est une saponine ou un acide aminé basique.

Le deuxième composant agit comme vecteur du premier composant, ce premier composant étant le principe actif. Ce deuxième composant a pour rôle essentiel de permettre au premier composant, le principe actif, de franchir la membrane des globules blancs.

De manière préférée, le deuxième composant est choisi dans le groupe constitué par les saponines et l'arginine.

De manière préférée, les saponines naturelles suivantes seront utilisées : les harpagosides, les ginsenosides, les saponines de type quillaja saponaria QS III et QS 21A.

Les associations préférées pour une utilisation au sens de la présente invention sont les suivantes : l'oligosaccharide est le raffinose sous forme libre ou dérivée et le deuxième composant une saponine ; l'oligosaccharide est le raffinose sous forme libre ou dérivée et le deuxième composant est l'arginine ; l'oligosaccharide est le stachyose sous forme libre ou dérivée et le deuxième composant est une saponine ; l'oligosaccharide est le stachyose sous forme libre ou dérivée et le deuxième composant est l'arginine.

Selon une forme préférée, ladite composition est telle que la concentration pondérale en oligosaccharide est comprise entre 0,01 % et 20 % par rapport à la masse totale de la composition, de préférence entre 0,01 % et 10 % par rapport à la masse totale de la composition. De manière préférée, la concentration du deuxième composant (saponines, arginine) est de préférence comprise entre 5 et 20 % en poids par rapport à la concentration en oligosaccharide, de préférence environ 10 % en poids par rapport à la concentration en oligosaccharide.
Cette composition contient en outre généralement au moins un excipient acceptable.

Les compositions utilisées selon la présente invention peuvent se présenter sous la forme d'une pommade, d'une crème, d'un collyre, d'une lotion, d'un spray destiné à la voie externe ou d'un spray pour inhalation. Sans vouloir être lié par une quelconque théorie, il semblerait que l'ingestion (boissons, tablettes, gélules) rende la composition inefficace, car vraisemblablement, les systèmes enzymatiques de l'estomac détruisant la partie sucrose de la molécule, la transformant en mélibiose. Or, les études *in vitro* ont montré que le mélibiose n'était pas actif sur les leucocytes.

Ces oligosaccharides constitués d'unités galactose reliées par une (ou des) liaisons de type a entre elles et à une unité sucrose, sont les substances de réserve d'un certain nombre de plantes (haricots, pois, soja,...) et sont surtout connus pour leur responsabilité dans le phénomène de flatulence chez l'homme. Par contre, aucune action directe pouvant présenter un intérêt thérapeutique (dermatologique) ou cosmétique n'avait jamais été à ce jour, mise en évidence.

Sans vouloir être lié à une quelconque théorie, ces molécules ont été testées sur l'activité anti-dégranulante des basophiles humains en partant de ridée que, l'organisme des mammifères ne possédant pas lés enzymes nécessaires à la dégradation des unités galactose associées en α, ces oligosaccharides pouvaient perturber la glycolyse des mastocytes c'est-à-dire la source d'énergie de la granulation et de la dégranulation.

Les oligosaccharides utilisés pour mettre en oeuvre la présente invention comportent de trois à cinq unités osidiques, avec au moins une unité D - galactose liée par son carbone 1 au carbone 6 d'une unité sucrose, au moyen d'une liaison α, selon la formule I suivante : 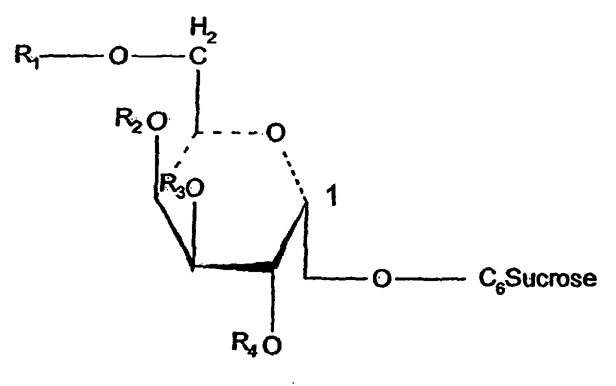 dans laquelle R₁, R₂, R₃ et R₄ représentent indépendamment l'un de l'autre un atome d'hydrogène, un groupe alkyle contenant de 1 à 10 atomes de carbone et possédant éventuellement au moins une insaturation, une fonction acide contenant de 1 à 10 atomes de carbone et possédant éventuellement au moins une insaturation, une fonction sulfate ou encore un ose qui peut se présenter sous forme libre ou dérivée.

En outre, l'unité sucrose peut aussi se présenter sous forme libre (c'est-à-dire un sucre dans lequel les fonctions alcools ne sont pas protégées) ou dérivée. Par ailleurs, cette unité sucrose semble indispensable à l'activité biologique puisque le mélibiose, testé dans les mêmes conditions d'expérience, ne présente strictement aucune activité sur les mastocytes.

Par « forme dérivée » au sens de la présente invention on entend que les fonctions alcools sont substituées par exemple par un groupe acétyle (CH₃CO)...

De manière préférée, on utilisera le stachyose et le raffinose.

Le stachyose est un tétraholoside dont la structure finale est :
α-D-Galactopyranosyl-(1-6)-α-D-Galactopyranosyl-(1-6)-α-D-Glucopyranosyl-(1-2)-β-D-Fructofuranoside.

Le stachyose et le raffinose correspondent à la formule présentée ci-dessous. 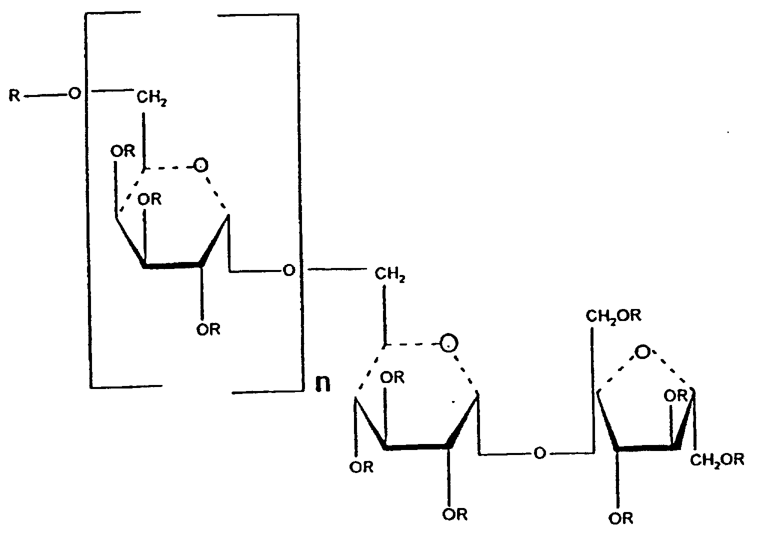 avec pour le stachyose R = H et n = 2 et pour le raffinose R = H et n = 1.

Les compositions utilisées selon l'invention peuvent être présentées sous forme de pommades, crèmes, collyre, sprays ou lotions pour l'administration locale, en association avec des excipients compatibles. Les excipients généralement utilisés pour préparer de telles compositions sont des liants, des conservateurs, des arômes.... Dans ces formes, le pourcentage pondéral en principe actif par rapport à la masse de la composition totale est compris entre 0,01 % et 20 %, de préférence 0,2 et 1 %. Des compositions pharmaceutiques d'inhalation peuvent aussi être préparées pour les traitements internes (asthme).

Afin de tester l'activité anti-dégranulanté ou anti-activatrice des oligosaccharides sur les basophiles humains par cytométrie de flux, des suspensions leucocytaires de sujets différents et sélectionnés comme étant de bons répondeurs à l'anti-igE ont été utilisées pour isoler, par sédimentation simple à 1g, les leucocytes. Ceux-ci sont pré-incubés pendant 30 minutes avec des dilutions successives du produit à tester de 10 à 0,01 mg/ml. Les basophiles sont alors stimulés par une concentration cible d'un anti-lgE humain et marqués par un mélange des anticorps anti-IgE FITC, anti-CD63PE, le CD63 étant un marqueur d'activation des basophiles humains. Les pourcentages d'activation ou d'inhibition sont alors calculés par le cytomètre de flux. L'activité biologique des oligosaccharides a été ainsi évaluée.

La structure chimique des molécules commerciales et des dérivés, en particulier celle du stachyose, a été vérifiée par RMN : RMN ¹H et ¹³C, RMN-2D (DQF-COSY, HMQC et HMBC).

Les mêmes tests biologiques ont été effectués sur des analogues structuraux comme le raffinose ou des oligosaccharides comportant une liaison α entre une unité galactose et une autre unité ose comme dans le melibiose.

Ces molécules fortement hydrosolubles n'ont que peu de chance de franchir les membranes liposolubles des mastocytes, ce que confirme les résultats des tests.

Cependant il est bien connu que les mastocytes ont une activité de pinocytose importante vis-à-vis de molécules chargés positivement (des bases donc) au pH physiologique.

Des essais ont donc été entrepris. Ces essais ont pour but de tester le pouvoir d'inhibition de la dégranulation de compositions contenant un premier composant -le principe actif- qui est un oligosaccharide choisi parmi le stachyose et le raffinose à différentes concentrations et un deuxième composant choisi dans le groupe fourni par l'arginine, la choline, le chlorure de calcium (CaCl₂) et la saponine. Le pourcentage d'inhibition a été calculé en renouvelant trois fois l'expérience.

Les résultats obtenus sont consignés dans le tableau 1, les abréviations suivantes ont été utilisées :

| | | | |
|---|---|---|---|
| "S" | pour Stachyose | R | pour Raffinose |
| "10" | pour 10 mg/ml | 1 | pour 1mg/ml |
| "0,1" | pour 0,1 mg/ml | "a" | pour arginine |
| "b" | pour choline | "c" | pour CaCl₂ |
| "Sp" | pour Saponine | "NT" | pour non testé. |

| Composition | S₁₀a | S₁a | S_{0,1}a | S₁₀b | S₁b | S_{0,1}b |
|---|---|---|---|---|---|---|
| % d'hinibition | 31 | 15 | 0 | 9 | 0 | 0 |
| Composition | S₁₀c | S₁c | S_{0,1}c | S₁₀sp | S₁sp | S_{0,1}sp |
| % d'inhibition | 6 | 0 | 0 | 76 | 0 | 0 |

| Composition | R₁₀a | R₁a | R_{0,1}a | R₁₀b | R₁b | R_{0,1} b |
|---|---|---|---|---|---|---|
| % d'hinibition | 19 | 5 | 0 | 2 | NT | NT |
| Composition | R₁₀c | R₁c | R_{0,1}c | R₁₀sp | R₁sp | R_{0,1}sp |
| % d'inhibition | 0 | NT | NT | 77 | 10 | 0 |

Au vu de ces résultats, il apparaît nettement que, aux concentrations testées, les compositions contenant de l'arginine et du stachyose ou du raffinose permettent d'inhiber la dégranulation ou la mise en route du système de granulation et donc d'inhiber la libération d'histamine, de bradykinine, de sérotonine et des facteurs chimiotactiques d'autres leucocytes.

Afin d'obtenir une plus grande inhibition des mastocytes, nous avons alors entrepris de favoriser la pénétration de ces molécules à travers les membranes en remplaçant l'arginine par de la saponine (Quillaja bark). Les résultats du tableau 1 montrent que des pourcentages d'inhibitions encore plus élevés sont alors obtenus.

L'utilisation des oligosaccharides selon la présente invention, en présence de molécules chargées positivement au pH physiologique (comme par exemple l'arginine) favorisant la pinocytose ou de molécules favorisant la pénétration membranaire (par exemple des saponines) permettent donc de bloquer ou réduire les activités biologiques des mastocytes, basophiles et éosinophiles chez les mammifères.

Aucune activité cytotoxique n'a été constatée au cours des études biologiques. Ce type de molécules peut donc être utilisé avec profit dans tous les domaines thérapeutiques (dermiques) et cosmétiques où les mastocytes et les basophiles humains (et par suite les éosinophiles) sont impliqués. Citons à titre d'exemple le prurit, les piqûres d'insectes, le psoriasis, la conjonctivite allergique, l'urticaire, l'eczéma et l'asthme.

Les exemples qui suivent ne limitent en aucun cas la portée de la présente invention.

### Exemples de formulation :

### Gel topique

| | |
|---|---|
| Oligosaccharide | 0,1 g |
| Arginine (ou saponines) | 0,01 g |
| Méthylcellulose | 3 g |
| Eau purifiée qsp | 100 g |
| Conservateurs, parfum | QS |

### Crème grasse

| | |
|---|---|
| Oligosaccharide | 0,1 g |
| Arginine (ou saponines) | 0,01 g |
| Eau purifiée | 5. ml |
| Corps gras qsp | 100 g |
| Conservateurs, parfum (appliquée telle quelle ou tulle gras) | QS |

### Emulsion

| | |
|---|---|
| Oligosaccharide | 0,2 g |
| Arginine (ou saponines) | 0,02 g |
| Monostéarate de PEG 1500 | 5 g |
| Monostéarate de PEG 300 | 2 g |
| Huile de paraffine fluide | 5 g |
| Eau purifiée qsp | 100 g |
| Conservateurs, parfum | QS |

### Inhalation

Une préparation destinée à l'inhalation est obtenue à partir du mélange suivant :

| | |
|---|---|
| Oligosaccharide | 100 mg |
| Arginine (ou saponines) | 10 mg |
| Excipient | 10 g |

A ce mélange, on ajoute un excipient classique pour formulation pour inhalation, comme par exemple l'acide oléique, ainsi qu'un gaz propulseur de type trichlorofluorométhane, dichlorodifiuorométhane.

### Crème grasse A (formule centésimale)

| Composants | % |
|---|---|
| Eau | 54,60 |
| PEG-6 et PEG-32 stéarate | 12,00 |
| Butyrospermum parkii | 10,00 |
| Raffinose | 10,00 |
| Glycérine | 7,00 |
| Paraffinum liquidum | 3,00 |
| Acide stéarique | 1,20 |
| Saponine | 1,00 |
| Méthylparaben | 0,20 |
| Imidazolidinyl urée | 0,20 |
| Bisabolol | 0,20 |
| Parfum | 0,20 |
| Propylparaben | 0,10 |
| BHT, BHA, propylgallate et acide citrique | 0,05 |
| Alcool benzylique, méthylchloroisothiazolinone et méthylisothiazolinone | 0,05 |

### Effet antiprurigineux sur le téqument récemment épidermisé du grand brûlé

En phase de cicatrisation, les grands brûlés souffrent de démangeaisons intenses, même parfois sous traitement lourd d'anti- histaminiques.

Cette étude, d'une durée d'un an, portant sur 33 patients a mis en évidence l'effet antiprurigineux puissant de la composition A dans cette indication.

### PROTOCOLE

Les patients souffrant de démangeaisons sont traités par une application de la composition A dont la concentration en raffinose est de 10%, ou par une application de la composition A' dont la concentration en raffinose est de 5%, ou encore par une application de la composition A" dont la concentration en raffinose est de 3% l'application étant attribuée par tirage au sort. Le pourcentage en saponine des compositions A' et A" représente un dixième de la concentration en raffinose.
En cas de deuxième zone à traiter parallèlement, le patient se voit appliquer en double aveugle et à son insu une composition considérée comme placebo sur cette deuxième zone contenant les mêmes excipients mais ne contenant ni raffinose, ni saponine.
Les trois tubes concentrés différemment en raffinose sont codés pour le patient et le personnel chargé de l'application.
Les applications sont renouvelées plusieurs fois par jour en cas de démangeaison et à la demande du patient et peuvent s'étaler sur plusieurs jours
Une fiche de suivi indique le temps d'action du ou des produits selon une échelle analogique du prurit de 0 à 10.

### RESULTATS ET CONCLUSIONS

Sur 33 cas traités, 30 ont mis en évidence une action du produit, 3 se sont révélés négatifs.
8 cas ont concerné deux zones d'application contre placebo.
Le temps moyen d'action de la composition A sur les 30 cas positifs se situe entre 2 et 5 mn, avec une durée d'action moyenne comprise entre 3 et 4 heures.
L'intensité du prurit initial estimé en moyenne à 7 sur l'échelle analogique, est à 0 dans les 30 cas après 15 mn.
Aucune différence statistiquement significative n'a pu être mis en évidence quant aux trois dosages en raffinose.
Contre placebo, on observe toujours les mêmes effets que ci-dessus pour la zone traitée avec la composition A.
Pour la zone traitée avec le placebo, l'intensité du prurit baisse légèrement de 0 à 5 mn pour remonter au niveau de l'intensité initiale au bout de 5 à 15mn. Ce phénomène transitoire bien connu est lié à l'hydratation de la peau.
Aucun phénomène d'intolérance n'a été signalé lors de l'étude en application simple ou répétée.
A signaler qu'un cas d'eczéma a régressé sous l'effet du traitement Sur les 3 cas négatifs, l'un est inexpliqué et les deux autres patients souffraient, pour l'un, de très forte inflammation et pour l'autre de sensation de cuisson, mais pas réellement de prurit.

## Revendications

1. Utilisation d'une association d'au moins deux composants pour la fabrication d'une composition pour le traitement d'au moins un symptôme choisi dans le groupe constitué par l'asthme, l'eczéma, le prurit, le psoriasis, la conjonctivite allergique, l'urticaire et les réactions aux piqûres d'insectes, les démangeaisons et en particulier les démangeaisons symptomatiques des grands brûlés ou à titre d'anti-inflammatoire, destinée à être administrée par voie topique exteme ou par inhalation, telle que le premier composant, qui est le principe actif, est un oligosaccharide de formule 1 contenant de trois à cinq unités osidiques et possédant au moins une unité D-galactose liée par une liaison α[1-6] avec une unité sucrose : 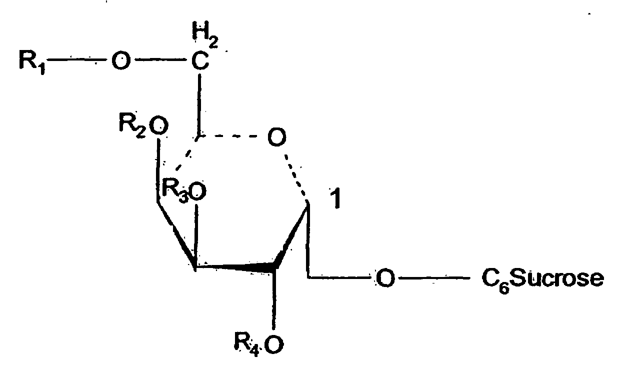 dans laquelle R₁, R₂, R₃ et R₄, indépendamment les uns des autres représentent un atome d'hydrogène, un groupe alkyle contenant de 1 à 10 atomes de carbone et possédant éventuellement au moins une insaturation, une fonction acide contenant de 1 à 10 atomes de carbone et possédant éventuellement au moins une insaturation, une fonction sulfate ou encore un ose et
le deuxième composant est une saponine ou un acide aminé basique.

2. Utilisation selon la revendication 1 telle que ledit oligosaccharide est choisi dans le groupe constitué par le raffinose et le stachyose sous forme libre ou dérivée.

3. Utilisation selon la revendication 1 ou 2 telle que le deuxième composant est choisi dans le groupe constitué par les saponines et l'arginine.

4. Utilisation selon la revendication 1 telle que l'oligosaccharide est le raffinose sous forme libre ou dérivée et le deuxième composant est une saponine.

5. Utilisation selon la revendication 1 telle que l'oligosaccharide est le raffinose sous forme libre ou dérivée et le deuxième composant est l'arginine.

6. Utilisation selon la revendication 1 telle que l'oligosaccharide est le stachyose sous forme libre ou dérivée et le deuxième composant est une saponine.

7. Utilisation selon la revendication 1 telle que l'oligosaccharide est le stachyose sous forme libre ou dérivée et le deuxième composant est l'arginine.

8. Utilisation selon l'une des revendications précédentes **caractérisée en ce que** la concentration pondérale en oligosaccharides est comprise entre 0,01 % et 20 % par rapport à la masse totale de la composition.

9. Utilisation selon l'une des revendications précédentes **caractérisée en ce que** la concentration du deuxième composant est comprise entre 5 % et 20 % en poids par rapport à la concentration en oligosaccharides.

10. Utilisation selon l'une des revendications précédentes **caractérisée en ce que** la concentration pondérale en oligosaccharides est comprise entre 0,01 % et 10% par rapport à la masse totale de la composition et la concentration du deuxième composant est environ 10 % en poids par rapport à la concentration en oligosaccharides.

11. Utilisation selon l'une des revendications précédentes telle que ladite composition se présente sous la forme d'une pommade, d'une crème, d'un collyre, d'une lotion, d'un spray destiné à la voie externe ou d'un spray pour inhalation.

## Claims

1. Use of a combination of at least two components for the manufacture of a composition for the treatment of at least one symptom chosen from the group consisting of asthma, eczema, pruritus, psoriasis, allergic conjunctivitis, urticaria, reactions to insect bites, and itching, and in particular the symptomatic itching of major burn sufferers, or as an antiinflammatory, which is intended to be administered topically and externally or by inhalation, such that the first component, which is the active principle, is an oligosaccharide of formula I containing from three to five osidic units and having at least one D-galactose unit linked via an α[1-6] bond with a sucrose unit: 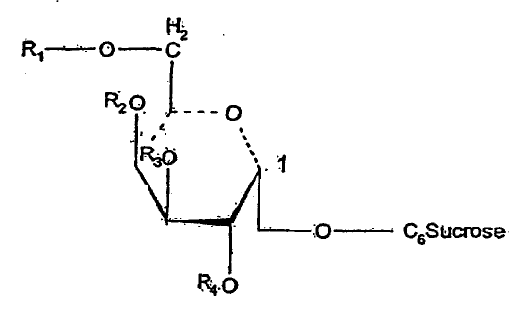 in which R₁, R₂, R₃ and R₄, independently of each other, represent a hydrogen atom, an alkyl group containing from 1 to 10 carbon atoms and optionally having at least one unsaturation, an acid function containing from 1 to 10 carbon atoms and optionally having at least one unsaturation, a sulphate function or a saccharide, and
the second component is a saponin or a basic amino acid.

2. Use according to Claim 1, wherein the said oligosaccharide, is chosen from the group consisting of raffinose and stachyose in free or derived form.

3. Use according to Claim 1 or 2, wherein the second component is chosen from the group consisting of saponins and arginine.

4. Use according to Claim 1, wherein the oligosaccharide is raffinose in free or derived form and the second component is a saponin.

5. Use according to Claim 1, wherein the oligosaccharide is raffinose in free or derived form and the second component is arginine.

6. Use according to Claim 1, wherein the oligosaccharide is stachyose in free or derived form and the second component is a saponin.

7. Use according to Claim 1, wherein the oligosaccharide is stachyose in free or derived form and the second component is arginine.

8. Use according to one of the preceding claims, **characterized in that** the weight concentration of oligosaccharides is between 0.01% and 20% relative to the total mass of the composition.

9. Use according to one of the preceding claims, **characterized in that** the concentration of the second component is between 5% and 20% by weight relative to the oligosaccharide concentration.

10. Use according to one of the preceding claims, **characterized in that** the weight concentration of oligosaccharides is between 0.01% and 10% relative to the total mass of the composition and the concentration of the second component is about 10% by weight relative to the oligosaccharide concentration.

11. Use according to one of the preceding claims, wherein the said composition is in the form of an ointment, a cream, eyedrops, a lotion, a spray intended for external use, or a spray for inhalation.

## Patentansprüche

1. Verwendung einer Assoziation von mindestens zwei Verbindungen für die Herstellung einer Zusammensetzung zur Behandlung mindestens eines Symptoms, das aus der Gruppe ausgewählt ist, die aus Asthma, Ekzem, Pruritus, Psoriasis, allergischer Konjunktivitis, Urtikaria und Reaktionen auf lnsektenstiche, Juckreizen und insbesondere symptomatischen Juckreizen bei schweren Verbrennungen besteht, oder als entzündungshemmendes Mittel, welche dazu bestimmt ist, auf äußerem topischen Weg oder durch Inhalation verabreicht zu werden, derart, dass die erste Verbindung, die der Wirkstoff ist, ein Oligosaccharid der Formel I ist, das drei bis fünf osidische Einheiten enthält und mindestens eine D-Galactose-Einheit besitzt, die durch eine α[1-6]-Bindung mit einer Saccharose-Einheit verbunden ist: 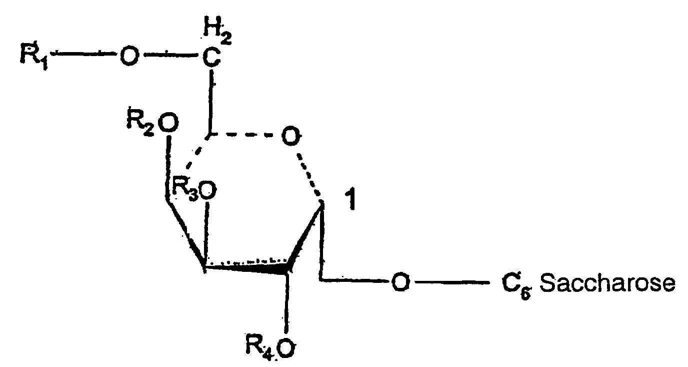 worin R₁, R₂, R₃ und R₄ unabhängig voneinander ein Wasserstoffatom, eine Alkylgruppe, die 1 bis 10 Kohlenstoffatome enthält und gegebenenfalls mindestens eine Unsättigung aufweist, eine Säurefunktion, die 1 bis 10 Kohlenstoffatome enthält und gegebenenfalls mindestens eine Unsättigung aufweist, eine Sulfatfunktion oder auch eine Ose sind, und
die zweite Verbindung ein Saponin oder eine basische Aminosäure ist.

2. Verwendung nach Anspruch 1, derart, dass das Oligosaccharid aus der Gruppe ausgewählt ist, die aus Raffinose und Stachyose in freier oder derivatisierter Form besteht.

3. Verwendung nach Anspruch 1 oder 2, derart, dass die zweite Verbindung aus der Gruppe ausgewählt ist, die aus Saponinen und Arginin besteht.

4. Verwendung nach Anspruch 1, derart, dass das Oligosaccharid Raffinose in freier oder derivatisierter Form ist und die zweite Verbindung ein Saponin ist.

5. Verwendung nach Anspruch 1, derart, dass das Oligosaccharid Raffinose in freier oder derivatisierter Form ist und die zweite Verbindung Arginin ist.

6. Verwendung nach Anspruch 1, derart, dass das Oligosaccharid Stachyose in freier oder derivatisierter Form ist und die zweite Verbindung ein Saponin ist.

7. Verwendung nach Anspruch 1, derart, dass das Oligosaccharid Stachyose in freier oder derivatisierter Form ist und die zweite Verbindung Arginin ist.

8. Verwendung nach einem der vorangehenden Ansprüche, **dadurch gekennzeichnet, dass** die Gewichtskonzentration an Oligosacchariden 0,01 % bis 20%, bezogen auf das Gesamtgewicht der Zusammensetzung, beträgt.

9. Verwendung nach einem der vorangehenden Ansprüche, **dadurch gekennzeichnet, dass** die Konzentration der zweiten Verbindung 5 bis 20 Gewichts-%, bezogen auf die Konzentration an Oligosacchariden, beträgt.

10. Verwendung nach einem der vorangehenden Ansprüche, **dadurch gekennzeichnet, dass** die Gewichtskonzentration an Oligosacchariden 0,01% bis 10%, bezogen auf das Gesamtgewicht der Zusammensetzung, beträgt und die Konzentration der zweiten Verbindung etwa 10 Gewichts-%, bezogen auf die Konzentration an Oligosacchariden, beträgt.

11. Verwendung nach einem der vorangehenden Ansprüche, derart, dass die Zusammensetzung in Form einer Salbe, einer Creme, von Augentropfen, einer Lotion, eines Sprays, das für den äußeren Weg bestimmt ist, oder eines Sprays zur Inhalation vorliegt.
